# EUROPÄISCHE PATENTSCHRIFT

(11) **EP 1 896 767 B1**
(45) Veröffentlichungstag und Bekanntmachung des Hinweises auf die Patenterteilung: **16.10.2013**
(21) Anmeldenummer: 06776568.5
(22) Anmeldetag: 02.08.2006
(51) Int. Cl.: F21S 8/00

(54) **OPERATIONSLEUCHTE**
OPERATING LUMINA
APPAREIL D'ÉCLAIRAGE DE SALLE D'OPÉRATION

(30) Priorität: 02.08.2005 DE 102005036275
(43) Veröffentlichungstag der Anmeldung: 12.03.2008
(73) Patentinhaber: Berchtold Holding GmbH, 78532 Tuttlingen (DE)
(72) Erfinder: SCHOLZ, Manfred, 78606 Seitingen-Oberflacht (DE)
(74) Vertreter: Manitz, Gerhart
(86) Internationale Anmeldenummer: PCT/EP2006/007655
(87) Internationale Veröffentlichungsnummer: WO 2007/014769

(56) Entgegenhaltungen:
- EP-A- 1 462 711
- WO-A-2005/012785
- DE-A1- 19 926 690
- US-A- 5 383 105

## Beschreibung

Die vorliegende Erfindung betrifft eine Operationsleuchte nach dem Oberbegriff des Anspruchs 1 (EP-A-1462 711).

Es sind auch Operationsleuchten bekannt, bei denen als Lichtquelle Halogenlampen oder Entladungslampen und als optische Mittel Reflektoren eingesetzt werden. Hierbei wird die sichtbare Strahlung der Lichtquelle in einer Richtung auf das zu beleuchtende Operationsfeld gerichtet, die nachfolgend als Hauptabstrahlrichtung bezeichnet wird.

Es ist die Aufgabe der Erfindung, eine Operationsleuchte zu schaffen, mit der die Leistungsfähigkeit des Operationsteams gesteigert werden kann.

Die Lösung dieser Aufgabe erfolgt durch die Merkmale des Anspruchs 1.

Erfindungsgemäß weist die Operationsleuchte zumindest ein Zusatzleuchtmittel, beispielsweise eine oder mehrere Leuchtdioden auf, dessen Intensitätsmaximum in einem Bereich von etwa 450 nm liegt, wobei eine Steuereinrichtung vorgesehen ist, mit der dieses zusätzliche Leuchtmittel unabhängig von der Lichtquelle, also beispielsweise weiteren Leuchtdioden, Halogenlampen oder Entladungslampen, ansteuerbar ist. Es wird die Erkenntnis ausgenutzt, dass Licht auf den Betrachter auch eine physiologische Wirkung hat. Durch Licht bestimmter Wellenlänge im blauen Farbbereich wird ein zweiter Anteil der Sehbahn aktiviert, der als energetischer Anteil den Stoffwechsel und die Hormondrüsen zur rhythmischen Tätigkeit aktiviert. Es ist bekannt, dass durch das Tageslicht die Ausschüttung eines Hormons (Melatonin) gehemmt wird, was zu einer Aktivierung des Kreislaufs dient, da Melatonin den Tag-Nacht-Rhythmus (circadianer Rhythmus) regelt. Durch Photosensoren im menschlichen Auge, die über die Netzhaut gleichmäßig verteilt sind und die ein Bildmuster nicht auflösen können, wird durch das blaue Licht die nächtliche Sekretion von Melatonin bewirkt. Nachdem Operationsteams häufig viele Stunden und oft auch Nachts unter Stress mit höchster Konzentration arbeiten müssen, kann durch eine passende Auswahl des Lichtspektrums der Chirurg so bei seiner Arbeit unterstützt werden, dass er auch während einer schwierigen Notoperation in der Nacht mit maximaler Konzentrationsfähigkeit arbeiten kann. Durch das zusätzliche Leuchtmittel mit Intensitätsmaximum im Bereich von etwa 450 nm lässt sich die Suppression der Melatoninsekretion anregen, so dass die Leistungsfähigkeit des Operationsteams gesteigert wird, auch wenn dieses nachts oder tagsüber über mehrere Stunden operieren muss.

Da das Zusatzleuchtmittel in einer Nebenabstrahlrichtung abstrahlt, die nicht auf das Operationsfeld gerichtet ist, lassen sich die gewünschten Effekte auf das Operationspersonal optimieren, da in diesem Fall mit Hilfe der Zusatzlichtquelle die Strahlung im Bereich von etwa 450 nm direkt in die Augen des Operationspersonals geleitet werden kann und somit keine Schwächung des Lichts durch Reflektionen an dem Operationsfeld erfolgt. Besonders gute Resultate können erzielt werden, wenn das Zusatzleuchtmittel in einer Nebenabstrahlrichtung abstrahlt, die unter einem Winkel von etwa 45 bis 135 ° zu der Hauptabstrahlrichtung verläuft. Die Abstrahlung kann auch seitlich oder nach oben, d.h. indirekt erfolgen. Ferner kann das Leuchtmittel auch in einem separaten Leuchtenkörper angeordnet werden.

Das Zusatzleuchtmittel ist durch die Steuereinrichtung automatisch in Abhängigkeit von bestimmten Parametern zuschaltbar. Derartige Parameter können beispielsweise die aktuelle Uhrzeit (Tageszeit) oder die aktuelle Betriebsdauer der Lichtquelle sein, aus der sich auf die bereits verstrichene Operationszeit zurückschließen lässt. Beispielsweise ist es möglich, die Abstrahlung des Zusatzleuchtmittels zu steigern, wenn die aktuelle Uhrzeit fortschreitet, d.h. wenn es zunehmend Nacht wird. Auch kann eine Steigerung erfolgen, wenn die Lichtquelle zunehmend eingeschaltet bleibt, d.h. die Operation beispielsweise über mehrere Stunden andauert. Hierdurch kann einerseits eine Ermüdung verhindert und andererseits sogar eine Leistungssteigerung bewirkt werden.

Vorteilhafte Ausführungsformen der Erfindung sind in der Beschreibung, des Zeichnung sowie den Unteransprüchen beschrieben.

Nach einer Ausführungsform wird ergänzend das Farbspektrum der Operationsleuchte so gesteuert, dass der gewünschte Wellenlängenbereich von etwa 450 nm mit erhöhter oder zunehmend erhöhter Intensität abgestrahlt wird.

Nach einer weiteren vorteilhaften Ausführungsform ist das Zusatzleuchtmittel auch bei ausgeschalteter Lichtquelle einschaltbar. Hierdurch lassen sich die gewünschten Wirkungen bereits vor der Operation erzielen, wenn das Operationspersonal vorbereitende Maßnahmen trifft. Auch kann die Wirkung des Zusatzleuchtmittels bei endoskopischen Operationen erfolgen. Wenn das Zusatzleuchtmittel bei eingeschalteter Lichtquelle ausschaltbar ist, lässt sich das Zusatzleuchtmittel schonen, wenn dessen Wirkung nicht benötigt wird, beispielsweise bei nur kurzen Eingriffen.

Nach einer weiteren vorteilhaften Ausführungsform kann das Zusatzleuchtmittel in einen Griff der Operationsleuchte integriert sein. Bei dieser Ausführungsform ist es nicht erforderlich, zur Aufnahme des Zusatzleuchtmittels den Leuchtenkörper zu verändern. Außerdern lässt sich das Zusatzleuchtmittel in dem Griff auf einfache Weise so anordnen, dass dieses in Richtung der Augen des Operationspersonals abstrahlt. Besonders vorteilhaft ist es, wenn das Zusatzleuchtmittel einen Diffusor aufweist, da sich in diesem Fall die Strahlung des Zusatzleuchtmittels bestmöglich im Operationssaal verteilt und somit gut in die Augen des Operationspersonals gelangen kann.

Nach einer weiteren vorteilhaften Ausführungsform kann die Steuerung einen Steuerausgang zur Ansteuerung eines externen Zusatzleuchtmittels aufweisen. Auf diese Weise lässt sich zusätzlich oder alternativ ein Zusatzleuchtmittel vorsehen, das nicht in die Operationsleuchte integriert ist. In diesem Fall kann als externes Zusatzleuchtmittel ein separates Lichtmodul vorgesehen werden oder das externe Zusatzleuchtmittel kann in eine stationäre Beleuchtung des Operationssaals integriert sein.

Nach einem weiteren Aspekt der Erfindung betrifft diese ein Verfahren zum Beleuchten eines Operationsraumes, wobei zumindest zeitweise die Strahlung zumindest eines Zusatzleuchtmittels direkt in Richtung der Augen des Operationspersonals gerichtet wird.

Nachfolgend wird die vorliegende Erfindung anhand einer vorteilhaften Ausführungsform und unter Bezugnahme auf die Zeichnung beschrieben.
Es zeigen:
- Fig. 1: eine teilweise geschnittene Seitenansicht einer Operationsleuchte;
- Fig. 2: eine teilweise geschnittene Seitenansicht einer weiteren Ausführungsform einer Operationsleuchte; und
- Fig. 3: eine vergrößerte Darstellung des Handgriffes der Operationsleuchte von Fig. 1.

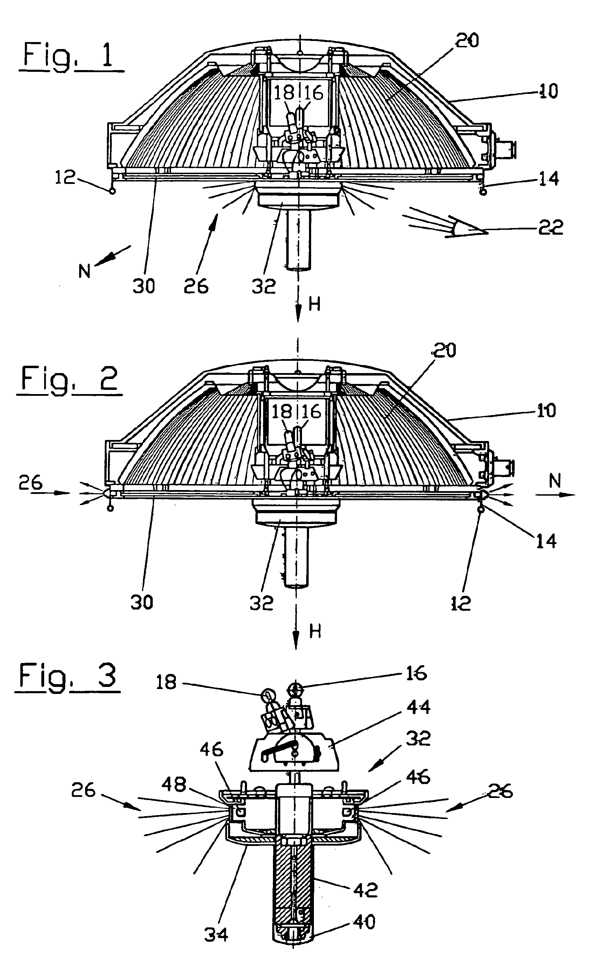

Die in Fig. 1 dargestellte Operationsleuchte weist einen haubenförmig ausgebildeten Leuchtenkörper 10 auf, der an seinem Außenumfang von einer Reling 12 umgeben ist, die über Stützelemente 14 an der Außenseite des Leuchtenkörpers 10 befestigt ist.

Im Inneren des Leuchtenkörpers 10 ist eine Lichtquelle in Form zweier wechselbarer Halogenlampen 16, 18 angeordnet, die über einen parabolförmigen Reflektor 20 ein Operationsfeld beleuchten. Hierbei ist der Reflektor 20 so ausgebildet, dass in der mit dem Pfeil H bezeichneten Hauptabstrahlrichtung ein schattenfreies Licht auf das Operationsfeld abgegeben wird.

Bei der in Fig. 1 dargestellten Ausführungsform ist im Inneren des Handgriffes 32 eine Zusatzleuchtmittel 26 in Form mehrerer Leuchtdioden vorgesehen, dessen Intensitätsmaximum in einem Bereich von etwa 450 nm liegt. Die Bandbreite des von dem Zusatzleuchtmittel abgestrahlten Spektrums liegt im Bereich von etwa 380 bis 520 nm. Das Zusatzleuchtmittel 16 strahlt in einer mit N bezeichneten Nebenabstrahlrichtung ab, die unter einem Winkel zur Hauptabstrahlrichtung H verläuft. Bei der in Fig. 1 dargestellten Ausführungsform beträgt dieser Winkel etwa 70°. Die Strahlung des Zusatzleuchtmittels 26 ist über den gesamten Umfang des Handgriffes 32 verteilt. Zur besseren Lichtverteilung sind optische Diffusoren vorgesehen, die eine diffuse Strahlung des Zusatzleuchtmittels erzeugen.

Wie Fig. 1 zeigt, gelangt die Strahlung des Zusatzleuchtmittels 26 in der Abstrahlrichtung N direkt und unmittelbar in das Auge 22 des Operationspersonals.

Zur Ansteuerung des Zusatzleuchtmittels 26 ist eine (nicht dargestellte) Steuereinrichtung vorgesehen, mit der das Zusatzleuchtmittel 26 unabhängig von der Lichtquelle 16, 18 ein- und ausschaltbar ist. Dies bedeutet, dass das Zusatzleuchtmittel 26 bei ausgeschalteter Lichtquelle 16, 18 einschaltbar ist und auch bei eingeschalteter Lichtquelle 16, 18 ausschaltbar ist.

Fig. 3 zeigt eine vergrößerte Darstellung des Handgriffes der Operationsleuchte von Fig. 1. Neben einer in einem Schaft 40 des Handgriffes angeordneten CCD-Kamera 42 weist der Handgriff 32 eine becherförmige Aufnahme 34 auf, an deren Unterseite der Schaft 40 befestigt ist. Die Aufnahme 34 ist an ihrer Oberseite offen und aus der offenen Oberseite steht eine Lampenhalterung 44 hervor, an der die wechselbaren Leuchtmittel 16, 18 befestigt sind. Der Handgriff weist auf bekannte Weise eine Einstellung für den Lichtfelddurchmesser auf.

Wie Fig. 3 zeigt, sind im Inneren der becherförmigen Aufnahme 34 und über deren Umfang verteilt mehrere Leuchtdioden 46 vorgesehen, die das Zusatzleuchtmittel 26 bilden. Vor den Leuchtdioden 46 ist jeweils eine Diffusorlinse 48 angeordnet, um diffuse Strahlung zu erzeugen.

An der Unterseite der Operationsleuchte ist eine transparente Abschlussscheibe 30 vorgesehen, in deren Mitte der Handgriff 32 angeordnet ist. Die Operationsleuchte selbst ist über nicht dargestellte Gelenkarme an einem Stativ oder einer Aufhängung befestigt.

Fig. 2 zeigt eine alternative Ausführungsform einer Operationsleuchte, wobei für gleiche Bauelemente gleiche Bezugszeichen verwendet sind.

Bei der in Fig. 2 dargestellten Operationsleuchte ist das Zusatzleuchtmittel 26 nicht in den Handgriff 32 sondern in den Leuchtenkörper 10 integriert. Hierbei sind Leuchtdioden über den Umfang der Operationsleuchte an deren Unterseite verteilt angeordnet, wobei die Nebenabstrahlrichtung N des Zusatzleuchtmittels bei dieser Ausführungsform etwa unter 90° zur Hauptabstrahlrichtung H verläuft. Auch bei dieser Ausführungsform sind vor den Leuchtdioden Diffusoren angeordnet.

Bei einer alternativen, nicht dargestellten Ausführungsform sind statt den Halogenlampen 16, 18 mehrere Leuchtdioden vorgesehen, die in unterschiedlichen Wellenlängen abstrahlen, um eine gewünschte Farbmischung zu erreichen.

Eine (nicht dargestellte) Steuereinrichtung dient zur Ansteuerung der Leuchtmittel der vorstehend beschriebenen Operationsleuchten, wobei das Zusatzleuchtmittel 26 unabhängig von den Leuchtmitteln 16, 18 zu- und abschaltbar ist.

Die Steuerung ist so ausgelegt, dass das herkömmliche OP-Licht unverändert bleibt, auch wenn die für die Strahlung von 450 nm benötigten Leuchtdioden mit erhöhter Intensität abstrahlen. Die Erhöhung der Strahlung im Bereich von 450 nm (nachfolgend Anregungsstrahlung genannt) kann durch einen Taster dauerhaft zugeschaltet werden. Alternativ ist mit Hilfe eines weiteren Tasters ein Automatikbetrieb auslösbar, bei dem die Anregungsstrahlung in Abhängigkeit von der aktuellen Uhrzeit und/oder der aktuellen Betriebsdauer der Operationsleuchte mit Hilfe eines vorgegebenen Programms automatisch zugeschaltet wird. Hierbei wird die Anregungsstrahlung mit zunehmender Betriebszeit der Operationsleuchte während einer Operation und mit weiter fortschreitender Tageszeit stetig erhöht. Zu diesem Zweck besitzt die Steuerung eine eingebaute Uhr und eine Zeitmesseinrichtung, die bei Einschalten der Operationsleuchte aktiviert wird, so dass auf die Dauer einer Operation rückgeschlossen werden kann.

### Bezugszeichenliste

- 10: Leuchtenkörper
- 12: Reling
- 14: Halterung
- 16, 18: Halogenlampen
- 20: Reflektor
- 22: Auge
- 26: Zusatzleuchtmittel
- 30: transparente Scheibe
- 32: Handgriff
- 34: Aufnahme
- 40: Schaft
- 42: elektronische Kamera
- 44: Halterung
- 46: Leuchtdioden
- 48: Diffusoren

- H: Hauptabstrahlrichtung
- N: Nebenabstrahlrichtung

## Patentansprüche

1. Operationsleuchte mit einem Leuchtenkörper und zumindest einer darin (10) angeordneten Lichtquelle (16, 18) und einem optischen Mittel (20), um die sichtbare Strahlung der Lichtquelle in einer Hauptabstrahlungsrichtung (H) auf ein Operationsfeld zu richten, und mit zumindest einem Zusatzleuchtmittel (26), dessen Intensitätsmaximum in einem Bereich von etwa 450 nm liegt, und einer Steuereinrichtung, mit der dieses Zusatzleuchtmittel (26) unabhängig von der Lichtquelle (16, 18) schaltbar ist, wobei
das Zusatzleuchtmittel in einer Nebenabstrahlrichtung abstrahlt, die nicht auf das Operationsfeld gerichtet ist,
**dadurch gekennzeichnet,**
**dass** das Zusatzleuchtmittel (26) durch die Steuereinrichtung anhand zumindest eines Parameters automatisch zuschaltbar ist, der aus folgender Gruppe ausgewählt ist: aktuelle Uhrzeit, aktuelle Betriebsdauer der Operationsleuchte.

2. Operationsleuchte nach Anspruch 1,
**dadurch gekennzeichnet,**
**dass** der Lichtstrom des Zusatzleuchtmittels (26) durch die Steuereinrichtung in Abhängigkeit von der aktuellen Betriebsdauer der Operationsleuchte automatisch veränderbar ist.

3. Operationsleuchte nach zumindest einem der vorstehenden Ansprüche,
**dadurch gekennzeichnet,**
**dass** das Zusatzleuchtmittel in einer Nebenabstrahlrichtung abstrahlt, die unter einem Winkel von etwa 45 - 135° zu der Hauptabstrahlrichtung verläuft.

4. Operationsleuchte nach zumindest einem der vorstehenden Ansprüche,
**dadurch gekennzeichnet,**
**dass** das Zusatzleuchtmittel (26) bei ausgeschalteter Lichtquelle (16, 18) einschaltbar ist.

5. Operationsleuchte nach zumindest einem der vorstehenden Ansprüche,
**dadurch gekennzeichnet,**
**dass** das Zusatzleuchtmittel (26) bei eingeschalteter Lichtquelle (16, 18) ausschaltbar ist.

6. Operationsleuchte nach zumindest einem der vorstehenden Ansprüche,
**dadurch gekennzeichnet,**
**dass** das Zusatzleuchtmittel (46) in einen Griff der Operationsleuchte integriert ist.

7. Operationsleuchte nach zumindest einem der vorstehenden Ansprüche,
**dadurch gekennzeichnet,**
**dass** das Zusatzleuchtmittel (26) in den Leuchtenkörper (10) integriert ist.

8. Operationsleuchte nach zumindest einem der vorstehenden Ansprüche,
**dadurch gekennzeichnet,**
**dass** das Zusatzleuchtmittel (26) einen Diffusor (48) aufweist.

9. Operationsleuchte nach zumindest einem der vorstehenden Ansprüche,
**dadurch gekennzeichnet,**
**dass** die Steuerung einen Steuerausgang zur Ansteuerung eines externen Zusatzleuchtmittels aufweist.

10. Operationsleuchte nach Anspruch 9,
**dadurch gekennzeichnet,**
**dass** ein separates Lichtmodul an den Steuerausgang angeschlossen ist.

11. Verfahren zum Beleuchten eines Operationsraumes, wobei mit einer Operationsleuchte mit zumindest einer in einem Leuchtenkörper angeordneten Lichtquelle und einem optischen Mittel sichtbare Strahlung der Lichtquelle in einer Hauptabstrahlungsrichtung auf ein Operationsfeld gerichtet wird, **dadurch gekennzeichnet,**
**dass** zumindest zeitweise die Strahlung zumindest eines Zusatzleuchtmittels, dessen Intensitätsmaximum in einem Bereich von etwa 450 nm liegt, direkt in Richtung der Augen des Operationspersonals gerichtet wird.

12. Verfahren nach Anspruch 11,
**dadurch gekennzeichnet,**
**dass** eine Operationsleuchte nach zumindest einem der Ansprüche 1 - 10 verwendet wird.

13. Verfahren nach Anspruch 11 oder 12,
**dadurch gekennzeichnet,**
**dass** das Zusatzleuchtmittel anhand zumindest eines Parameters automatisch eingeschaltet wird, der aus folgender Gruppe ausgewählt ist: aktuelle Uhrzeit, aktuelle Betriebsdauer der Operationsleuchte.

14. Verfahren nach zumindest einem der Ansprüche 11 bis 13,
**dadurch gekennzeichnet,**
**dass** der Lichtstrom des Zusatzleuchtmittels in Abhängigkeit von der aktuellen Betriebsdauer der Operationsleuchte automatisch verändert wird.

## Claims

1. A surgical lamp having a lamp body (10) and at least one light source (16, 18) arranged therein and having an optical means (20) to direct the visible radiation of the light source in a main radiation direction (H) onto a surgical field, and having at least one additional illuminant (26) whose intensity maximum is in a region of approximately 450 nm, and having a control device with which this additional illuminant (26) can be switched independently of the light source (16, 18),
wherein the additional illuminant radiates in a secondary radiation direction which is not directed to the surgical field,
**characterized in that**
the additional illuminant (26) can be switched in automatically by the control device with reference to at least one parameter which is selected from the following group: current time, current operating duration of the surgical lamp.

2. A surgical lamp in accordance with claim 1,
**characterized in that**
the luminous flux of the additional illuminant (26) is automatically variable by the control device in dependence on the current operating duration of the surgical lamp.

3. A surgical lamp in accordance with at least one of the preceding claims,
**characterized in that**
the additional illuminant radiates in a secondary radiation direction which extends at an angle of approximately 45 - 135° to the main radiation direction.

4. A surgical lamp in accordance with at least one of the preceding claims,
**characterized in that**
the additional illuminant (26) can be switched on when the light source (16, 18) is switched off.

5. A surgical lamp in accordance with at least one of the preceding claims,
**characterized in that**
the additional illuminant (26) can be switched off when the light source (16, 18) is switched on.

6. A surgical lamp in accordance with at least one of the preceding claims,
**characterized in that**
the additional illuminant (46) is integrated into a handle of the surgical lamp.

7. A surgical lamp in accordance with at least one of the preceding claims,
**characterized in that**
the additional illuminant (26) is integrated into the lamp body (10).

8. A surgical lamp in accordance with at least one of the preceding claims,
**characterized in that**
the additional illuminant (26) has a diffuser (48).

9. A surgical lamp in accordance with at least one of the preceding claims,
**characterized in that**
the control has a control output for the control of an external additional illuminant.

10. A surgical lamp in accordance with claim 9,
**characterized in that**
a separate light module is connected to the control output.

11. A method for the illumination of an operating room, wherein visible radiation of a light source is directed in a main radiation direction onto a surgical field using a surgical lamp having at least one light source arranged in a lamp body and an optical means,
**characterized in that**
the radiation of at least one additional illuminant whose intensity maximum is in a region of approximately 450 nm is directed directly in the direction of the eyes of the surgical personnel at least at times.

12. A method in accordance with claim 11,
**characterized in that**
a surgical lamp in accordance with at least one of the claims 1-10 is used.

13. A method in accordance with claim 11 or claim 12,
**characterized in that**
the additional illuminant is switched on automatically with reference to at least one parameter selected from the following group: current time, current operating duration of the surgical lamp.

14. A method in accordance with at least one of the claims 11 to 13, **characterized in that** the luminous flux of the additional illuminant is automatically varied in dependence on the current operation duration of the surgical lamp.

## Revendications

1. Lampe pour site d'opération comprenant un corps de lampe (10) et au moins une source de lumière (16, 18) agencée dans celui-ci, et un moyen optique (20) afin de diriger le rayonnement visible de la source de lumière dans une direction de rayonnement principale (H) vers un champ opératoire, et comprenant au moins un agent d'éclairage supplémentaire (26), dont le maximum d'intensité est dans une plage d'environ 450 nm, et comprenant un moyen de commande avec lequel cet agent d'éclairage supplémentaire (26) peut être commuté indépendamment de la source de lumière (16, 18), l'agent d'éclairage supplémentaire envoyant un rayonnement dans une direction de rayonnement annexe qui n'est pas dirigée vers le champ opératoire, **caractérisée en ce que** l'agent d'éclairage supplémentaire (26) est susceptible d'être commuté automatiquement en marche par le moyen de commande à l'aide d'au moins un paramètre qui est choisi parmi le groupe suivant : heure actuelle, durée de service actuelle de la lampe pour site d'opération.

2. Lampe pour site d'opération selon la revendication 1,
**caractérisée en ce que** le flux lumineux de l'agent d'éclairage supplémentaire (26) est modifiable automatiquement par le moyen de commande en fonction de la durée de service actuelle de la lampe pour site d'opération.

3. Lampe pour site d'opération selon l'une au moins des revendications précédentes,
**caractérisée en ce que** l'agent d'éclairage supplémentaire envoie un rayonnement dans une direction de rayonnement annexe qui s'étend sous un angle d'environ 45 à 135° par rapport à la direction de rayonnement principale.

4. Lampe pour site d'opération selon l'une au moins des revendications précédentes,
**caractérisée en ce que** l'agent d'éclairage supplémentaire (26) est susceptible d'être mis en marche lorsque la source de lumière (16, 18) est arrêtée.

5. Lampe pour site d'opération selon l'une au moins des revendications précédentes,
**caractérisée en ce que** l'agent d'éclairage supplémentaire (26) est susceptible d'être arrêté lorsque la source de lumière (16, 18) est mise en marche.

6. Lampe pour site d'opération selon l'une au moins des revendications précédentes,
**caractérisée en ce que** l'agent d'éclairage supplémentaire (46) est agencé dans une poignée de la lampe pour site d'opération.

7. Lampe pour site d'opération selon l'une au moins des revendications précédentes,
**caractérisée en ce que** l'agent d'éclairage supplémentaire (26) est intégré dans le corps de lampe (10).

8. Lampe pour site d'opération selon l'une au moins des revendications précédentes,
**caractérisée en ce que** l'agent d'éclairage supplémentaire (26) comprend un diffuseur (48).

9. Lampe pour site d'opération selon l'une au moins des revendications précédentes,
**caractérisée en ce que** la commande comprend une sortie de commande pour le pilotage d'un agent d'éclairage supplémentaire externe.

10. Lampe pour site d'opération selon la revendication 9,
**caractérisée en ce qu'**un module lumineux séparé est raccordé à la sortie de commande.

11. Procédé pour éclairer une salle d'opération, dans lequel, avec une lampe pour site d'opération comprenant au moins une source de lumière agencée dans un corps de lampe et un moyen optique, un rayonnement visible de la source de lumière est dirigé dans une direction de rayonnement principale vers un champ opératoire, **caractérisé en ce que** le rayonnement d'au moins un agent d'éclairage supplémentaire, dont le maximum d'intensité est dans une plage d'environ 450 nm, est au moins temporairement dirigé en direction des yeux du personnel opératoire.

12. Procédé selon la revendication 11,
**caractérisé en ce que** l'on emploie une lampe pour site d'opération selon l'une au moins des revendications 1 à 10.

13. Procédé selon la revendication 11 ou 12,
**caractérisé en ce que** l'agent d'éclairage supplémentaire est commuté automatiquement en marche à l'aide d'au moins un paramètre qui est choisi parmi le groupe suivant : heure actuelle, durée de fonctionnement actuelle de la lampe pour site d'opération.

14. Procédé selon l'une au moins des revendications 11 à 13,
**caractérisé en ce que** le flux de lumière de l'agent lumineux supplémentaire est modifié automatiquement en fonction de la durée de fonctionnement actuel de la lampe pour site d'opération.
